# EUROPEAN PATENT APPLICATION

(11) **EP 3 747 455 A1**
(43) Date of publication of application: **09.12.2020**
(21) Application number: 18903887.0
(22) Date of filing: 29.11.2018
(51) Int. Cl.: A61K 38/16, A61P 27/02

(54) **CORNEAL EPITHELIAL CELL CHEMOTAXIS PROMOTER**

(30) Priority: 30.01.2018 JP 2018013491
(71) Applicant: Shioda Life Science Inc., Minato-ku Tokyo 106-0031 (JP)
(72) Inventor: SHIODA Seiji, Tokyo 106-0032 (JP); NAKAMACHI Tomoya, Toyama-shi, Toyama 930-0887 (JP)
(74) Representative: dompatent von Kreisler Selting Werner - Partnerschaft von Patent- und Rechtsanwälten mbB
(86) International application number: PCT/JP2018/043912
(87) International publication number: WO 2019/150734

(57) **Abstract**

A problem to be solved by the present invention is to provide a novel agent that promotes chemotaxis of corneal epithelial cells. The problem is solved by an agent that promotes chemotaxis of corneal epithelial cells, comprising PACAP, PACAP derivatives, or a pharmaceutically acceptable salt thereof, as an active ingredient. The agent that promotes chemotaxis of corneal epithelial cells of the present invention preferably comprise 1 or more peptides belonging to a group PACAP, PACAP derivatives, or a pharmaceutically acceptable salt thereof. Concentration of PACAP, PACAP derivatives, or a pharmaceutically acceptable salt thereof is particularly preferably 1 × 10⁻¹³ mol/L or more and 1 × 10⁻⁷ mol/L or less. An agent that promotes chemotaxis of corneal epithelial cells of the present invention may be widely used as pharmaceuticals and the like for the improvement of symptoms of eye diseases such as dry eyes.

## Description

### TECHNICAL FIELD

The present invention relates to an agent that promotes chemotaxis of corneal epithelial cells, comprising PACAP, PACAP derivatives, or a pharmaceutically acceptable salt thereof, as an active ingredient.

### BACKGROUND ART

PACAP (Pituitary Adenylate Cyclase-Activating Polypeptide) is a type of neuropeptide. PACAP is a peptide consisting of 27 or 38 amino acid residues. PACAP has previously been reported for use as a neuritogenic agent, an anti-inflammatory agent, a chronic lung disease therapeutic agent, an eye disease therapeutic agent, and a tear secretagogue (Patent Document 1-5, Non-Patent Document 1-2).

Patent Document 6 describes the growth-promoting effects of corneal epithelial cells by PACAP administration. In addition, Patent Document 6 describes that restoration of a corneal epithelial was accelerated by administering PACAP to an eye of a rabbit with detached corneas, in comparison with controls.

However, the detailed mechanism for the growth-promoting effect of corneal epithelial cells by PACAP administration and the effect of PACAP administration to corneal endothelial cells have not been elucidated. Further researched and developments such as the effect of PACAP in the cornea are required.

### PRIOR ART DOCUMENTS

### PATENT DOCUMENTS

Patent Document 1: JP 2001-226284A
Patent Document 2: JP 2004-224775A
Patent Document 3: JP 2004-315436A
Patent Document 4: JP 2006-306770A
Patent Document 5: JP 2009-269818A
Patent Document 6: WO 2005/102375

### NON PATENT DOCUMENTS

Non-Patent Document 1: Vaudry D, et al., Pituitary Adenylate Cyclase-Activating Polypeptide and Its Receptors :20 years after the Discovery. Pharmacol Rev 2009; 61(3) ; 283-357.
Non-Patent Document 2: Nakamachi T, et al., PACAP suppresses dry eye signs by stimulating tear secretion. Nat Commun. 2016;7:12034

### SUMMARY OF THE INVENTION

### PROBLEMS TO BE SOLVED BY THE INVENTION

The present invention has been accomplished in view of the above-mentioned background art, and a problem thereof is to provide a new application of PACAP.

### MEANS FOR SOLVING THE PROBLEMS

As a result of an intense consideration to solve the above challenges, the inventors found that PACAP is involved in the corneal epithelial cell-growth-promoting effect as well as in the chemotactic effect of the corneal epithelial cell, leading to the completion of the present invention.

That is, the present invention is directed to An agent that promotes chemotaxis of corneal epithelial cells, comprising PACAP, PACAP derivatives, or a pharmaceutically acceptable salt thereof, as an active ingredient.

### EFFECTS OF THE INVENTION

According to the present invention, it is possible to provide an agent that promotes chemotaxis of corneal epithelial cells. The agent can be used for preventing or treating ocular diseases such as corneal disorders. Therefore, the above problem can be solved.

### BRIEF DESCRIPTION OF DRAWINGS

[Fig. 1] Fig. 1 is a photograph showing the results of the cell scratch assay.
[Fig. 2] Fig. 2 is a graph showing the area ratios of wounded sites 18 h after adding PACAP.
[Fig. 3] Fig. 3 is a graph showing the results of measuring the numbers of cells at 1 day after the addition of PACAP (left) and at 4 days after the addition of PACAP by MTT-assay.
[Fig. 4] Fig. 4 is a photograph showing dividing cells with BraU labeling.
[Fig. 5] Fig. 5 is a graph showing quantitative results of the numbers of dividing cells by BraU labeling.
[Fig. 6] Fig. 6 is a graph showing the area ratios of wounded sites at 18 hours after adding PACAP alone, or PACAP and AraC.
[Fig. 7] Fig. 7 is a photograph showing the results of RT-PCRs in the brain, cornea, and corneal epithelia.
[Fig. 8] Fig. 8 is a photograph showing PAC1R immunopositive reactions in the cornea.
[Fig. 9]

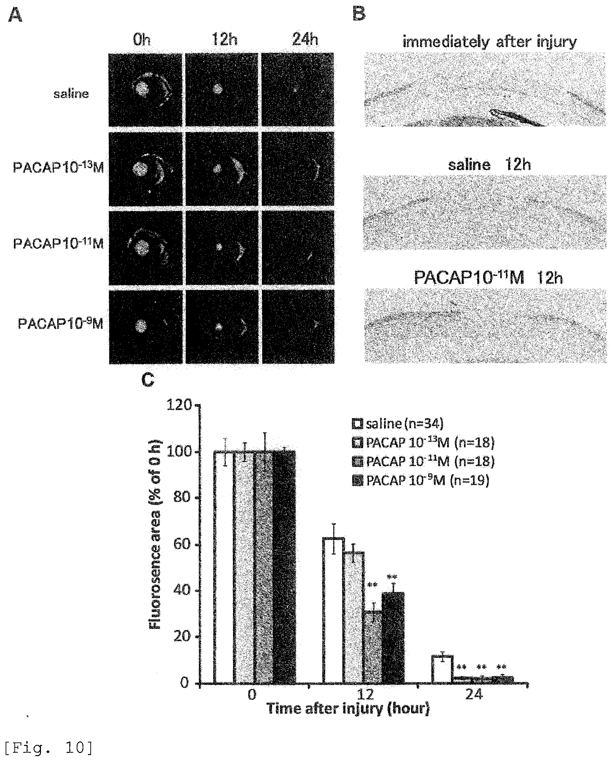

   Fig. 9A is a photograph showing the fluorescence image of injured sites of corneas.
   Fig. 9B is a photograph showing HE staining images at 0 hours and 12 hours after injuring.
   Fig. 9C is a graph showing changes in the injured sites of corneas (fluorescent areas) after administration of PACAP.
[Fig. 10]

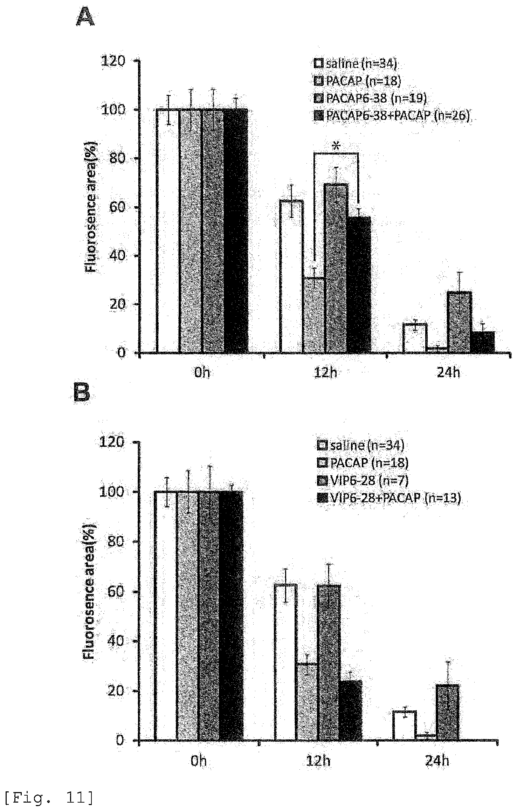

   Fig. 10A is a graph showing changes in the injured sites of corneas (fluorescent areas) after administration of PACAP6-38.
   Fig. 10B is a graph showing changes in the injured sites of corneas (fluorescent areas) after administration of VIP6-28.

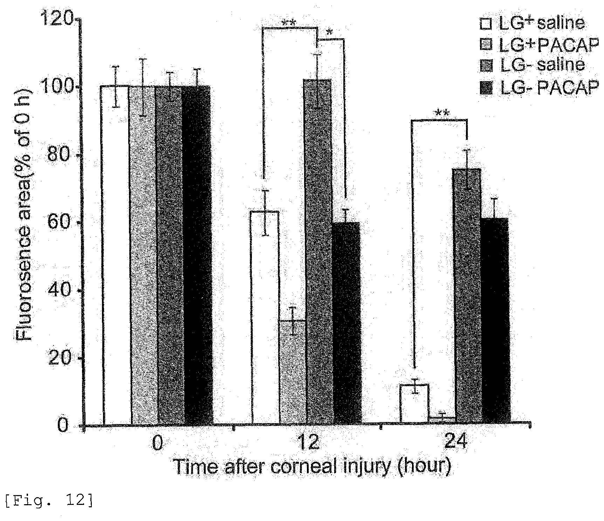

[Fig. 11] Fig. 11 is a graph showing changes in the injured sites of corneas (fluorescent areas) of mice with lacrimal glands removed.
[Fig. 12]

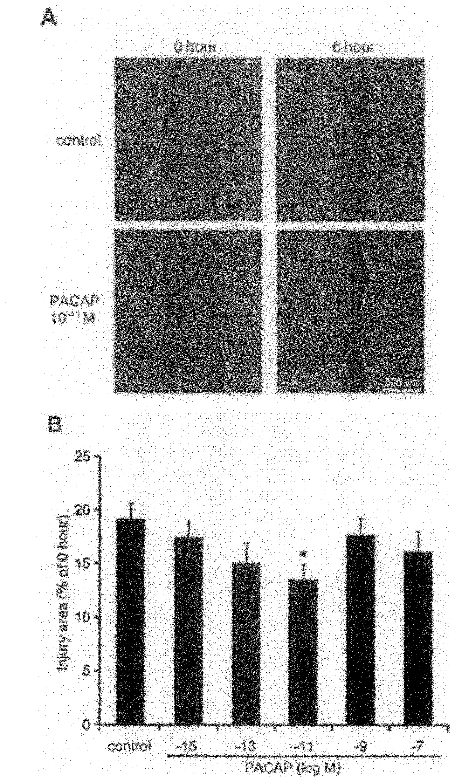

   Fig. 12A is a photograph showing the result of in vitro scratch assay on monolayer sheets of human corneal cultured cells.
   Fig. 12B is a graph showing changes in the scratch sites at 6 hours after scratching the cells.

### EMBODIMENTS TO CARRY OUT THE INVENTION

In the following, the present invention is explained, but the present invention is not limited by the following specific embodiments, and can be optionally changed within the range of the technical thought of the present invention.

In the present invention, an agent that promotes chemotaxis of corneal epithelial cells comprises PACAP, PACAP derivatives, or a pharmaceutically acceptable salt thereof, as an active ingredient.

PACAP (Pituitary Adenylate Cyclase-Activating Polypeptide) is a neuropeptide consisting of 27 or 38 amino acid residues. PACAP is strongly expressed in the central and peripheral nervous systems and widely distributed in peripheral tissues such as testis, adrenal gland, and intestine.

In the present invention, it is preferable to use PACAP38 (PACAP composed of 38 amino acid residues) for reasons such as sufficiently exhibiting the effect of the present invention. The amino acid sequence of PACAP38 is described, for example, in Patent Document 6.

PACAP receptors include VPAC receptors (VPAC1 receptors, VPAC2 receptors) and PAC1 receptors. VPAC receptors also bind to VIP (Vasoactive intestinal polypeptide) with comparable affinity and promote the production of cAMP. PAC1 receptors bind selectively to PACAP and, besides cAMP production, also activate phosphatidylinositol turnover and MAP-kinase.

The present invention is the first to find that PACAP administration promotes chemotaxis of corneal epithelial cells, as shown in Examples and others.

As illustrated in the Examples, the present invention also relates to an agent that acts on a PAC1 receptor (PAC1R) in the cornea, comprising PACAP, PACAP derivatives, or a pharmaceutically acceptable salt thereof, as an active ingredient.

In the present invention, it is preferred that an agent that promotes chemotaxis of corneal epithelial cells comprises 1 or more peptides belonging to a group consisting of "PACAP and pharmaceutically acceptable salts thereof".

Known peptide synthesis methods may be used for the synthesis of PACAP used in the present invention, and are not limited to certain synthesis methods.

Azide method, acid chloride method, acid anhydride method, mixed acid anhydride method, DCC method, active ester method, carboimidazole method, oxidation-reduction method, and DCC-additive method are examples of synthetic methods of PACAP used in the present invention.

These synthetic methods can be applied to either solid-phase synthesis or liquid-phase synthesis.

Examples of pharmaceutically acceptable salts of PACAP include salts with alkaline metals such as sodium and potassium; salts with alkaline earth metals such as calcium and magnesium; salts with aluminum; salts with inorganic bases such as ammonium; salts with organic bases such as trimethylamine, pyridine, picoline, ethanolamine, diethanolamine, triethanolamine, dicyclohexylamine and N,N-dibenzylethylenediamine; salts with inorganic acids such as hydrochloric acid, hydrobromic acid, nitric acid, sulfuric acid and phosphoric acid; salts with organic acids such as formic acid, acetic acid, trifluoroacetic acid, fumaric acid, oxalic acid, tartaric acid, lactic acid, maleic acid, citric acid, succinic acid, malic acid, methanesulfonic acid, benzenesulfonic acid and p-toluenesulfonic acid; salts with polymerized acids such as tannic acid, carboxy methylcellulose, polylactic acid and polyglycolic acid.

In the present invention, an agent that promotes chemotaxis of corneal epithelial cells comprises PACAP, PACAP derivatives, or a pharmaceutically acceptable salt thereof, as an active ingredient.

"PACAP derivatives" mean, for example, a derivative in which some amino acids in a polypeptide structure of PACAP have been deleted or substituted, or a derivative in which another amino acid has been inserted into a polypeptide structure of PACAP.

Also, those in which a modifying group such as a sugar chain is added to a polypeptide structure of PACAP are examples of "PACAP derivatives".

PACAP derivatives have the effect of promoting the chemotaxis of corneal epithelial cells.

Pharmaceutically acceptable salts, synthetic methods, and the like of PACAP derivatives may be used or applied in the same manner as those of PACAP described above.

PACAP, PACAP derivatives, or a pharmaceutically acceptable salt thereof are preferably extracted or synthesized, and are preferably isolated or purified.

The amount of PACAP, PACAP derivatives, or a pharmaceutically acceptable salt thereof contained in an agent that promotes chemotaxis of corneal epithelial cells, is not particularly limited. A suitable amount is selected according to the purpose.

The total amount of PACAP, PACAP derivatives, or a pharmaceutically acceptable salt thereof contained in an agent that promotes chemotaxis of corneal epithelial cells, is preferably 10⁻¹³ mol/L to 10⁻⁷ mol/L, more preferably 10⁻¹² mol/L to 10⁻⁸ mol/L, further preferably 10⁻¹¹ mol/L to 10⁻⁹ mol/L, particularly preferably 10⁻¹¹ mol/L to 10⁻¹⁰ mol/L.

As will be described later, particularly when used as an ophthalmic agent such as an eye drop, an eye wash, an ophthalmic ointment, or the like, it is desirable that the ophthalmic agent contains " PACAP, PACAP derivatives, or a pharmaceutically acceptable salt thereof" within the above range.

In the present invention, an agent that promotes chemotaxis of corneal epithelial cells comprises "PACAP, PACAP derivatives, or a pharmaceutically acceptable salt thereof", as an active ingredient. As described above, even if the concentration of the active ingredient is extremely low, the effect of the present invention described above is exhibited.

Any one of PACAP, PACAP derivatives, or a pharmaceutically acceptable salt thereof may be contained in an agent that promotes chemotaxis of corneal epithelial cells, or 2 or more of them may be used in combination. When 2 or more of them are used in combination, the above amount (range) is a total amount thereof.

When 2 or more of them are used in combination, there is no particular limitation on the content ratio of each compounds contained in the agent that promotes chemotaxis of corneal epithelial cells.
An appropriate content ratio is selected according to the purpose.

Also, in the present invention, an agent that promotes chemotaxis of corneal epithelial cells may contain "other components" in addition to PACAP, PACAP derivatives, or a pharmaceutically acceptable salt thereof.

There is no particular limitation on the "other components" in the agent that promotes chemotaxis of corneal epithelial cells described above. The "other ingredients" may be selected accordingly to the purpose within the scope that does not compromise the effect of the present invention. For example, pharmaceutically acceptable carriers, other pharmaceutically active ingredients, and the like may be mentioned as the "other ingredients".

There is no particular limitation on such carriers. Such carriers may be appropriately selected depending on the dosage form and the like described later. Further, there is no particular limitation on the amount of the "other components" in the agent that promotes chemotaxis of corneal epithelial cells. A suitable amount is selected according to the purpose.

In the present invention, a dosage form of an agent that promotes chemotaxis of corneal epithelial cells is not particularly limited. The dosage form may be appropriately selected depending on, for example, a desired administration method as described later.

Examples of the dosage form are, tablets, coated tablets, granules, powders, capsules, injections (solvents, suspensions, and the like), eye drops, eye detergents, ointments, eye ointments, patches, gels, creams, powders for external use, sprays, inhalation powders, and the like.

In the present invention, within the range not impairing the effect of the present invention, other additives commonly used in making may be added when making an agent that promotes chemotaxis of corneal epithelial cells into the above dosage form.

Examples of the additives are, a binder, a disintegrant, a lubricant, a colorant, a flavoring and flavoring agent, a buffer, a stabilizer, a pH regulator, an isotonizing agent, a preservative, and the like.

In the present invention, for the reason that the effect of the present invention can be sufficiently exhibited, and the like, the dosage form of an agent that promotes chemotaxis of corneal epithelial cells of the is preferably an ophthalmic agent such as an eye drop, an eye wash, or an eye ointment. The dosage form is more preferably an eye drop.

An ophthalmic agent may be formulated into the desired dosage form according to known methods.

In the present invention, an agent that promotes chemotaxis of corneal epithelial cells can be used, for example, by administering to an individual in need of prevention or treatment of corneal disorders.

In the present invention, animals to be administered an agent that promotes chemotaxis of corneal epithelial cells are not particularly limited, and examples thereof include humans; mice; rats; monkeys; domestic animals such as horses, cows, pigs, goats, chickens, and the like; pets such as cats, dogs, rabbits, and the like; and the like.

There is no particular limitation in the method of administering the agent that promotes chemotaxis of corneal epithelial cells described above. Depending on the dosage form and the like of the agent that promotes chemotaxis of corneal epithelial cells described above, the administration method may be appropriately selected. Examples of the administration method are oral administration, injection into the blood, and ocular administration.

There is no particular limitation in the dosage of the agent that promotes chemotaxis of corneal epithelial cells. The dosage may be appropriately selected according to the age, the weight, the degree of the desired effect, and the like of the individual to be administered.
For example, a daily dosage to an adult is preferably from 1mg to 30g, more preferably from 10mg to 10g, and particularly preferably from 100mg to 3g.

There is also no particular limitation in the timing of administration of an agent that promotes chemotaxis of corneal epithelial cells. An appropriate timing is selected according to the purpose. For example, the agent may be administered prophylactically, or may be administered therapeutically.

In the present invention, when the dosage form of an agent that promotes chemotaxis of corneal epithelial cells is an eye drop, the eye drop may be instilled preferably 1 time to 10 times per day (particularly preferably 2 times to 5 times per day). The amount of instillation per time is preferably 10 µL to 50 µL.

In the present invention, an agent that promotes chemotaxis of corneal epithelial cells promote damage healing effects of corneal cells by promoting the chemotaxis of corneal epithelial cells. Accordingly, an agent that promotes chemotaxis of corneal epithelial cells of the present invention is useful as a therapeutic agent for corneal cell damages, and is useful, for example, for preventing and/or treating corneal disorders and corneal ulcers.

Causes of the corneal disorders include, for example, corneal damage, reduction of tear fluid due to dry eye, foreign matter, contact lens wear, infection, corneal refractive surgery, cataract surgery, Sjoegren's syndrome, conjunctivitis, and the like.

In the present invention, since PACAP receptors express on corneal endothelial cells, an agent that promotes chemotaxis of corneal epithelial cells may be involved in the growth effects and/or chemotactic promoting effects of corneal endothelial cells.

### EXAMPLES

Hereinafter, the present invention will be explained more specifically by showing Examples; but the present invention is not limited to them unless beyond its scope.

"%" means "mass %", and "M" means "mol/L", unless otherwise stated.

### <Materials and methods>

### [PACAP]

As PACAP, PACAP38 (Peptide Research Institute, Osaka) was used.

### [Cell culture]

Normal human corneal epithelial cells HCEC-II (manufactured by Kurabo Industries Ltd.) were cultured in an OcuLifeBM medium containing OcuLifeLifeFactor (both manufactured by Kurashiki Spinning Co., Ltd.) at 37 °C, in the presence of 5 % CO₂.

### [Analysis of cell growth ability]

HCEC-II cells were seeded in 96-well plates and cultured for 24 h. Subsequently, PACAP alone, or PACAP and AraC (cytarabine) were added. AraC (cytarabine) is a cytostatic agent. After the addition of these agents, the cell growth ability was analyzed by measuring the number of live cells from the value of absorbance at 570nm using MTT cell growth kit I (manufactured by Roche Life Science Co., Ltd.). The reference wavelength was set at 650nm.

### [Scratch assay]

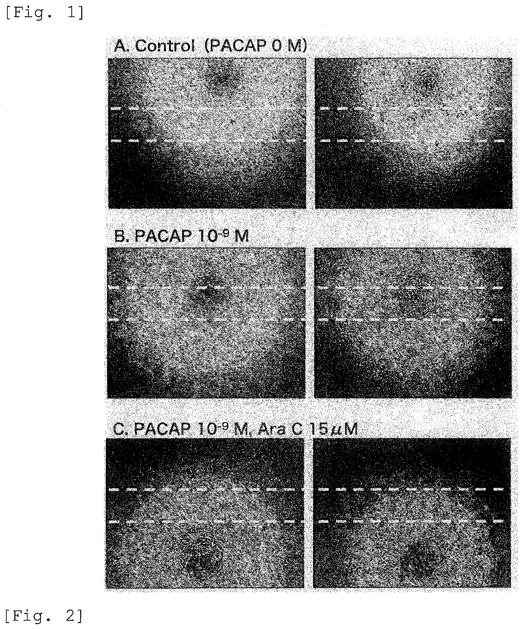

Wound-healing-promoting effects of PACAP were evaluated by scratch assay. HCEC-II cells were seeded in 24-well plates, cultured, and then wounded by a CELL Scratcher scratch stick (manufactured by Asahi Glass Co., Ltd.) (between a dotted line and a dotted line in Fig. 1).

Immediately after wounding, PACAP38 alone or PACAP38 and AraC were added, and cells infiltrating the wounded area were observed.

### [BrdU labeling]

Post-wounding dividing cells were observed by labeling with BrdU Labeling and Detection Kit I (Roche).

### [Image analysis].

Outcomes of scratch assay and BrdU labeling were evaluated as following. Images were acquired by BZ-X700 (Keyence Inc.), and then quantitatively analyzed using the analysis applications (hybrid cell counts, macro cell counts) (Keyence Inc.).

### Example 1

### <Wound-healing-promoting effects of PACAP on corneal cells>

To examine whether PACAP has wound-healing effects on corneal cells, a scratch assay was performed using HCEC-II cells. The results showed that, in the presence of 10⁻⁹ M PACAP at 4 days post-wounding, more cellular infiltrates were observed at the wounded site (between a dotted line and a dotted line in Fig. 1) compared to controls (Fig. 1A, B).

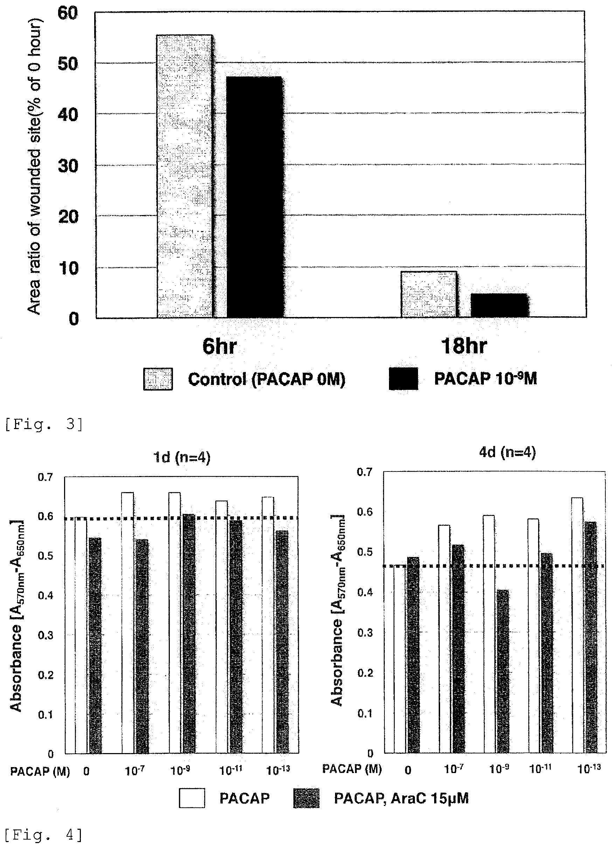

Also, similar experiments were performed to quantify the area of the wounded site at each time. The area of the wounded site was reduced in the presence of 10⁻⁹ M PACAP compared to controls (Fig. 2). This result suggested that PACAP has wound-healing-promoting effects on corneal cells.

### Example 2

### <Cell-growth promoting activity of PACAP>

Scratch assay results suggested that PACAP had wound-healing-promoting effects on corneal cells. The effects of PACAP are either due to 1) promoting cell growth, 2) promoting cell chemotaxis, or 3) promoting both cell growth and chemotaxis. To elucidate which of these could be caused, cell growth on days 1 and 4 after adding various concentrations of PACAP (10⁻⁹ M to 10⁻¹³ M) were analyzed by MTT-assay.

As shown in Fig. 3, at 1 day after the addition of PACAP, cells grew at any concentration relative to cells that were added no PACAP. At 4 days after the addition of PACAP, the effect was remarkable.

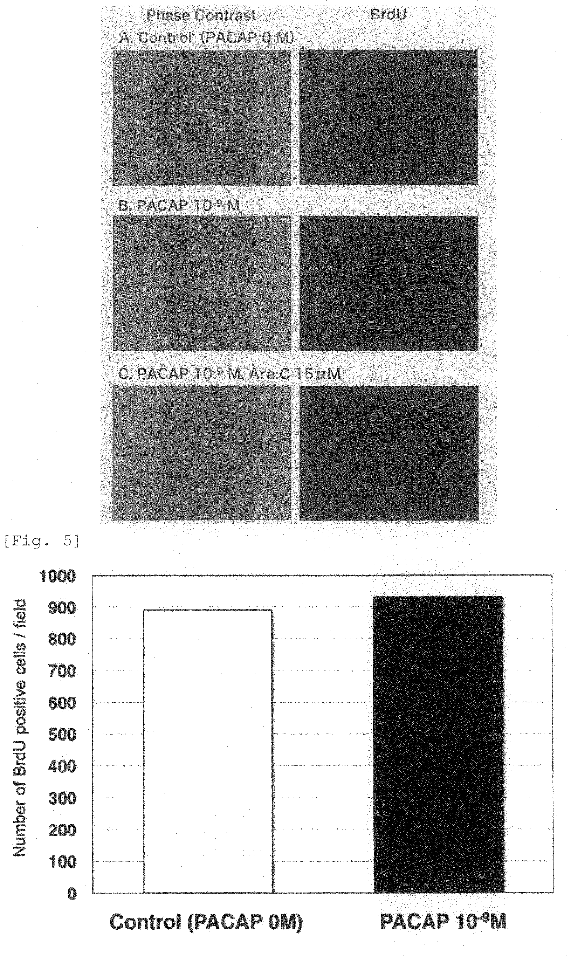

Fig. 4 shows dividing cells by BrdU labeling 18 hours after adding PACAP. In measuring number of dividing cells, an increase of number of BrdU positive cells were observed by adding PACAP (Figs. 4A, 4B and 5).

These results indicate that one part of the wound-healing-promoting effect of PACAP is due to its cell-growth-promoting effect.

### Example 3

### <Cell-chemotaxis-promoting activity of PACAP>

Next, the inventors analyzed whether PACAP had a cell-chemotaxis-promoting activity on corneal cells in the presence of AraC (cytarabine), a cell growth inhibitor.

First, the inventors investigated the concentrations of AraC, which inhibits growth of HCEC-II cells. At 15 µM of AraC concentration, the cell-growth-promoting effects of PACAP were almost completely suppressed. For this reason, subsequent experiments were performed under the condition that AraC concentration was 15 µM (Fig. 1, Fig. 4C) .

Scratch assay was performed using HCEC-II cells. Cell-infiltration was less in the presence of 10⁻⁹ M PACAP and 15 µM AraC than in the presence of 10⁻⁹ M PACAP alone, but increased more than in controls (Fig. 1A to C).

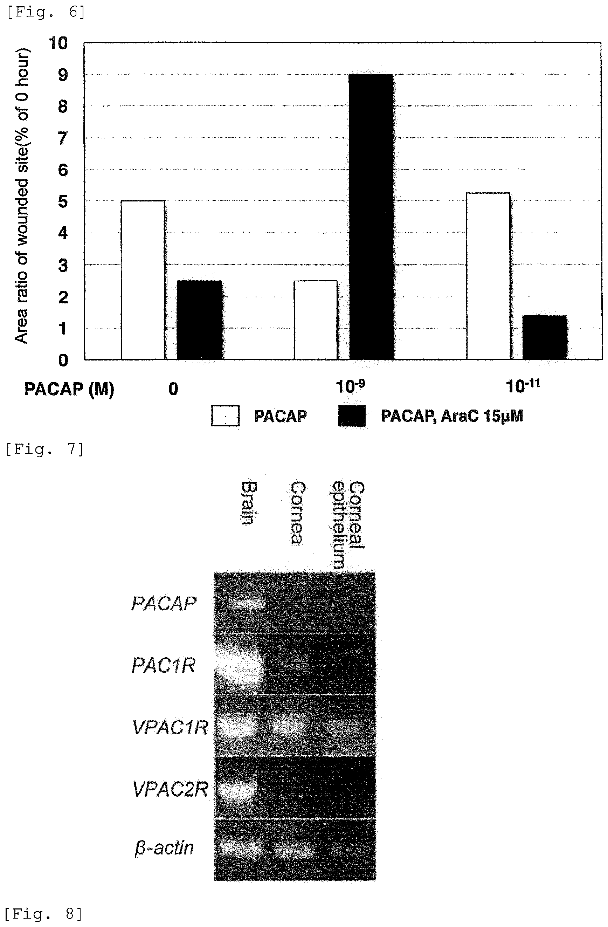

Similar experiments were performed to quantify the area of the wounded site at 18 h. When 10⁻⁹ M PACAP was added, the area of the wounded site was increased in the presence of 15 µM AraC compared to the control. On the contrary, when 10⁻¹¹ M PACAP was added, the area of the wounded site was reduced even in the presence of 15µM AraC (Fig. 6).

These results reveal that PACAP is not only effective in promoting cell growth but also effective in promoting cell chemotaxis.

### <Summary of Examples 1-3>

It was suggested that PACAP had wound-healing-promoting effects on corneal cells.

It was revealed that both cell-growth-promoting effect and cell-chemotaxis-promoting effect of PACAP were concerned in this effect.

### Example 4

### <Study of the expression of PACAP and PACAP receptors in corneas of mice>

The expression of PACAP and PACAP receptors in corneas was evaluated by RT-PCR method and fluorescent immunostaining method.

The inventors first extracted total RNAs from a cornea, a corneal epithelium, and a brain of a mouse using Trisol (registered mark) (Invitrogen) and generated cDNA by reverse-transcription reactions. Subsequently, PCRs were performed using specific primers for PACAP, PAC1 receptor (PAC1R), VPAC1 receptor (VPAC1R), and VPAC2 receptor (VPAC2R) genes, and bands were confirmed by agarose gel electrophoresis.

The results of RT-PCR method confirmed the expression of PAC1R and VPAC1R mRNA, but not of PACAP and VPAC2R mRNA, in the corneal and the corneal epithelia (Fig. 7). In Fig. 7, "Brain" is a positive control in which PACAP and its receptors are all expressed, and "β actin" is used as an internal standard.

Next, the cornea of a mouse was extracted and was fixed with formalin solution, and paraffin embedding was performed according to a conventional method. Paraffin sections of 4 µm thickness were prepared, and deparaffinization and blocking with 5 % horse normal serum were performed. Immunostaining was performed using a rabbit anti PAC1R antibody as the primary antibody and an Alexa546 labeled anti-rabbit IgG as the secondary antibody, and a nucleus was further stained by DAPI. A section that did not react with the primary antibody were used as a negative control. After the slides were encapsulated, PAC1R immunopositive reactions were observed by fluorescent microscopy.

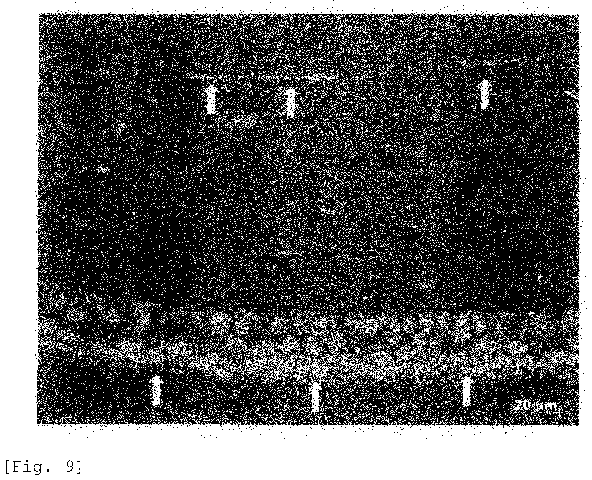

Results of fluorescent immunostaining are shown in Fig. 8. In Fig. 8, the upper layer of shows a corneal endothelial layer, and the lower layer shows a corneal epithelial layer. PAC1R immunopositive reactions were observed in the base of the corneal epithelial and in the corneal endothelium (e.g., arrows in Fig. 8). No positive reactions were observed in the negative control that did not react with the primary antibody.

These results suggest that PACAP may act on PAC1R present in a corneal epithelium and in a corneal endothelium.

### Example 5

### <Study of healing-promoting effects of PACAP instillation on model mice with injured corneas>

To study the healing effects of PACAP instillation on an injured site of a corneal, the inventors created model mice with injured corneas. Injured sites were created by marking a cornea of a mouse (C57BL/6J, male, 8-12 weeks old) with a 1-mm-diameter biopsy punch and detaching corneal epithelia within the marks with tweezers. At the injured sites, 2 µL of 10⁻⁹ M PACAP, 10⁻¹¹ M PACAP, 10⁻¹³ M PACAP, or saline was instilled every 2 hours for a total of seven times up to 12 hours later. In addition, the injured sites were visualized by instilling 2 µL of 0.1 % fluorescein (a fluorescent colorant) in a total of 3 times after 0, 12, and 24 hours, and photographing was performed.

In addition, eyeballs at after 0 and 12 hours were removed, and tissue sections were made after paraffin embedding, followed by hematoxylin and eosin staining (HE staining). From the captured fluorescent images, by using image analysis software (ImageJ), the area and the outer periphery of injured sites of corneas were measured when saline was instilled and when PACAP of the respective concentrations were instilled.

Fig. 9A shows changes in the size of injured sites of corneas at 0, 12, and 24 hours after injuring. The injured sites are indicated by circular fluorescence regions in the central of corneas. The injured sites became smaller over time in the saline group. In addition, the injured sites of the PACAP-instilled group became smaller than those of the saline-instilled group at 12 and 24 hours, which means healing was accelerated.

Fig. 9B shows the outcome of HE-staining at 0 and 12 hours after injuring. HE-stained image immediately after injury showed that only the corneal epithelium (dark gray layer in upper part of Fig. 9B) was removed at the injured site, and no injury was observed in the corneal stroma propria (layer just below the corneal epithelial layer in upper part of Fig. 9B). 12 hours after instillation of saline (saline 12h), the corneal epithelium was slightly restored (Fig. 9B, middle part). 12 hours after instillation of PACAP (PACAP 12h), the corneal epithelium was restored (Fig. 9B, lower part).

Fig. 9C shows the result of fluorescent area measured from the fluorescent image of the injured site of the cornea. The area of the injured site of the cornea immediately after injury to the cornea (0 hour) is set as 100 %. The smaller the number on the vertical axis, the more effective the healing of the injured site of the cornea.

At 12 hours after injuring of the cornea, the area of the injured site of the cornea was significantly smaller in the 10⁻¹¹ M PACAP-instilled group and the 10⁻⁹ M PACAP-instilled group than in the saline group. At 24 hours after injuring of the cornea, the area of the injured site of the cornea was significantly smaller in PACAP-instilled group of all concentrations (** : p < 0.01 (in comparison with saline group)). 10⁻¹¹ M was most effective concentration of PACAP.

The periphery of the injured site of the cornea showed the same tendency as the area of the injured site of the cornea. At 12 hours after injuring, the injured area was significantly smaller in the 10⁻¹¹ M PACAP-instilled group and the 10⁻⁹ M PACAP-instilled group than in the saline group. At 24 hours after injuring, the injured area was significantly smaller in PACAP-instilled group of all concentrations (not shown in figures).

### Example 6

### <Healing-promoting effects of instillation of PACAP receptor antagonists on model mice with injured corneas>

Next, to investigate which types of receptors are involved in healing-promoting effects of injured corneas by PACAP, the inventors performed an instillation experiment of PACAP receptor antagonists (PACAP6-38, VIP6-28). PACAP6-38 is an antagonist of PAC1R and VPAC2R. VIP6-28 is an antagonist of VPAC1R and VPAC2R.

In addition to the same operations as the instillation experiment in Example 5, PACAP6-38 (antagonist) (10⁻⁹ M) or VIP6-28 (antagonist) (10⁻⁹ M) was instilled immediately before instillation of PACAP and saline. Instillation of PACAP and saline ware performed every 2 hours.

Following four groups were created; the saline-only-instilled group, the PACAP-only-instilled group, the group instilled saline and an antagonist simultaneously, the group instilled PACAP and an antagonist simultaneously.

Results of simultaneous instillation experiments of PACAP receptor antagonists (PACAP6-38 and VIP6-28) are shown in Fig. 10. Fig. 10A is the result of PACAP6-38, and Fig. 10B is the result of VIP6-28. In Fig. 10, "saline" indicates the saline-only-instilled group. "PACAP" indicates the PACAP-only-instilled group. "PACAP6-38" or "VIP6-28" indicates the group instilled saline and an antagonist simultaneously. "PACAP6-38+PACAP" or "VIP6-28+PACAP" indicates the group instilled PACAP and an antagonist simultaneously.

As well as Fig. 9C, the area of the injured site of the cornea immediately after injury to the cornea (0 hour) is set as 100 %. The smaller the number on the vertical axis, the more effective the healing of the injured site of the cornea.

Compared with the PACAP-only-instilled group, in the group instilled PACAP and PACAP6-38 simultaneously, PACAP-induced healing of injured corneas was significantly suppressed at 12 hours after injuring of the cornea (* : p < 0.05). However, healing of injured corneas was not suppressed in the group instilled PACAP and VIP6-28 simultaneously. Also, administration of PACAP6-38 and the saline simultaneously, and administration of VIP6-28 and the saline simultaneously gave similar results to that of administration of the saline alone.

### Example 7

### <Corneal healing effects of PACAP on mice with lacrimal glands removed>

It has previously been reported that, by instilling PACAP, PACAP acts as a tear-promoting agent via a lacrimal gland. Thus, corneal healing effects of PACAP may be caused by an increase in tear volume due to PACAP. To confirm the corneal healing pathway of PACAP, the inventors injured corneas of mice with lacrimal glands removed, and verified the effects of PACAP.

First, mice with lacrimal glands removed were generated. Under inhalation anesthesia with sevoflurane, the extraorbital lacrimal glands and lacrimal ducts of mice were removed. Both lacrimal glands were removed after the lacrimal ducts were removed to expose the intraorbital lacrimal glands. Then the skin was sutured and the mice were kept warm until awaking from anesthesia. The amount of tear secretion in mice before and after removal of lacrimal glands was confirmed by the cotton thread method.

The amount of tear secretion after removal of lacrimal glands reduced by approximately 90 % compared with that of before removal of lacrimal glands. Instillation of PACAP did not promote tear secretion of mice with lacrimal glands removed (not shown in figures).

Fig. 11 is a graph showing the measurement result of the area of injured sites of corneas (fluorescent area) of upon administration of saline or PACAP at 0, 12, and 24 hours after injuring of corneas of mice. The method and the frequency of administration of saline or PACAP were the same as in Example 5.

The area of the injured site of the cornea immediately after injury to the cornea (0 hour) is set as 100 %. The smaller the number on the vertical axis, the more effective the healing of the injured site of the cornea.

In Fig. 11, "LG + saline" indicates the result of saline administration to mice without lacrimal glands removed. "LG + PACAP" indicates the result of PACAP administration to mice without lacrimal glands removed. "LG - saline" indicates the result of saline administration to mice with lacrimal glands removed. "LG - PACAP" indicates the result of PACAP administration to mice with lacrimal glands removed. The concentration of PACAP is 10⁻¹¹ M.

In "LG - saline", the area of injured sites of corneas (fluorescent area) did not become smaller compared to the result of "LG + saline". Thus, it was found that the corneal healing effects were suppressed by removing the lacrimal gland (** : p < 0.01).

On the other hand, in "LG - PACAP", the area of injured sites of corneas (fluorescent area) became significantly smaller compared to the result of "LG - saline" at 12 hours after injuring of the cornea (* : p < 0.05). This result suggests that PACAP is directly involved in the corneal healing effects, rather than via tear secretions.

### Example 8

### <In vitro scratch assay on monolayer sheets of human corneal cultured cells>

To evaluate the corneal healing effects of PACAP in vitro, a scratch assay was performed using a human corneal epithelial cell line. A SV40-human corneal epithelial cell line (HCEC) was purchased from the Human Science Research Resource Bank (Health Science Research Resources Bank). Cells were cultured in a medium containing DMEM, 1 × antibiotic-antifungal agent (manufactured by Thermo Fisher Scientific Co., Ltd.), and 10% fetal bovine serum (FBS). Cultures were performed at 37 °C in the presence of 5 % CO ₂.

In invasion assays, a floating HCEC with 10⁶ cells was seeded in 12-well plates. After 2 days of incubation, the confluent cell layer was cross-shaped wounded with plastic sticks. PBS or various types of PACAP (final concentrations: 10⁻¹⁵ M, 10⁻¹³ M, 10⁻¹¹ M, 10⁻⁹ M, and 10⁻⁷ M) were added to 10 µL wells. Scratch sites (wounded sites) were photographed by Nicon Biostation CT (Nikon Co., Ltd.) at regular intervals. Areas of the scratch sites were measured by CL-Quant software (made by Nikon Inc.).

The results are shown in Fig 12A and B. FIG. 12A shows scratch sites at 0 and 6 hours after scratching (wounding) the cells. Centrals part of each pictures, where cells are not dense, are the scratch sites.

Fig. 12B shows the areas of the scratch sites at 6 hours after scratching the cells. The area of the scratch site immediately after the scratch (0 hour) is set as 100%. The smaller the number on the vertical axis, the more effective the healing of the scratch site.

In Fig. 12, "control" indicates the result of "PBS-treated group (control group)".

At 6 hours after scratching the cell, the scratch site was significantly reduced when 10⁻¹¹ M of PACAP was administered into the cell, compared to the control group (PBS-treated group).

Therefore, it was suggested that PACAP exerts healing effects on human corneal epithelial cells in vitro.

### INDUSTRIAL APPLICABILITY

An agent that promotes chemotaxis of corneal epithelial cells of the present invention may be widely used as pharmaceuticals and the like for the improvement of symptoms of eye diseases such as corneal disorders.

## Claims

1. An agent that promotes chemotaxis of corneal epithelial cells, comprising PACAP, PACAP derivatives, or a pharmaceutically acceptable salt thereof, as an active ingredient.

2. The agent that promotes chemotaxis of corneal epithelial cells according to claim 1, wherein concentration of PACAP, PACAP derivatives, or a pharmaceutically acceptable salt thereof is 1 × 10⁻¹³ mol/L or more and 1 × 10⁻⁷ mol/L or less.
